# EUROPEAN PATENT SPECIFICATION

(11) **EP 2 510 356 B1**
(45) Date of publication and mention of the grant of the patent: **31.10.2018**
(21) Application number: 10836527.1
(22) Date of filing: 07.12.2010
(51) Int. Cl.: G01N 33/68

(54) **DIAGNOSTIC METHOD TO IDENTIFY WOMEN AT RISK FOR PRETERM DELIVERY**
DIAGNOSEVERFAHREN ZUR IDENTIFIKATION DES RISIKOS VON FRÜHGEBURTEN
PROCÉDÉ DE DIAGNOSTIC POUR IDENTIFIER DES FEMMES QUI PRÉSENTENT UN RISQUE D'ACCOUCHEMENT AVANT TERME

(30) Priority: 08.12.2009 US 267693 P
(43) Date of publication of application: 17.10.2012
(73) Proprietor: Cedars-Sinai Medical Center, Los Angeles, CA 90048 (US)
(72) Inventor: EQUILS, Ozlem, Sherman Oaks California 91411 (US); SIMMONS, Charles, F. Jr., Los Angeles California 90024 (US)
(74) Representative: HGF Limited
(86) International application number: PCT/US2010/059248
(87) International publication number: WO 2011/071893

(56) References cited:
- WO-A1-2007/112514
- WO-A1-2008/056114
- WO-A2-2009/134452
- US-A- 5 545 616
- US-A1- 2004 014 063
- US-A1- 2004 014 063
- US-A1- 2007 161 125
- US-A1- 2009 226 397
- US-A1- 2010 137 263
- US-B2- 7 232 661
- US-B2- 7 790 463
- J AMORY ET AL: "Increased tumor necrosis factor-[alpha] production after lipopolysaccharide stimulation of whole blood in patients with previous preterm delivery complicated by intra-amniotic infection or inflammation", AMERICAN JOURNAL OF OBSTETRICS AND GYNECOLOGY, vol. 185, no. 5, 1 November 2001 (2001-11-01), pages 1064-1067, XP055057802, ISSN: 0002-9378, DOI: 10.1067/mob.2001.117637
- KALINKA J ET AL: "Interleukin-1 beta and interleukin-1 receptor antagonist gene polymorphisms and the risk of spontaneous preterm delivery in the population of Polish women", ARCHIVES OF PERINATAL MEDICINE, BLACKHORSE SCIENTIFIC PUBLISHERS, LTD, PL , vol. 14, no. 4 1 January 2008 (2008-01-01), pages 33-36, XP008155809, ISSN: 1505-0580 Retrieved from the Internet: URL:http://www.ptmp.pl/archives/apm/14-4/A PM144-7-Kalinka.pdf
- CURRY ET AL.: 'First-trimester maternal plasma cytokine levels, pre-pregnancy body mass index, and spontaneous preterm delivery.' ACTA OBSTET GYNECOL SCAND. vol. 88, no. 3, January 2009, pages 332 - 342, XP008155818
- KALINKA ET AL.: 'Interleukin-1 beta and interleukin-1 receptor antagonist gene polymorphisms and the risk of spontaneous preterm delivery in the population of Polish women.' ARCH PERINATAL MED vol. 14, no. 4, 2008, pages 33 - 36, XP008155809
- BUSSEN ET AL.: 'Thyroid autoantibodies in euthyroid non-pregnant women with recurrent spontaneous abortions.' HUMAN REPRODUCTION vol. 10, no. 11, November 1995, pages 2938 - 2940, XP008155821
- SKOGSTRAND ET AL.: 'Simultaneous measurement of 25 inflammatory markers and neurotrophins in neonatal dried blood spots by immunoassay with xMAP technology.' CLIN CHEM. vol. 51, no. 10, October 2005, pages 1854 - 1866, XP008155811
- ROMERO ET AL.: 'The natural interleukin-1 receptor antagonist prevents interleukin-1-induced preterm delivery in mice.' AM J OBSTET GYNECOL. vol. 167, no. 4, October 1992, pages 1041 - 1045, XP008155820
- M. Marzi ET AL: "Characterization of type 1 and type 2 cytokine production profile in physiologic and pathologic human pregnancy", Clinical & Experimental Immunology, vol. 106, no. 1, 1 October 1996 (1996-10-01), pages 127-133, XP055109739, ISSN: 0009-9104, DOI: 10.1046/j.1365-2249.1996.d01-809.x
- WETTA L ET AL: "168: Impaired anti-inflammatory response in women with a prior spontaneous preterm birth", AMERICAN JOURNAL OF OBSTETRICS & GYNECOLOGY, MOSBY, ST LOUIS, MO, US, vol. 199, no. 6, 1 December 2008 (2008-12-01), page S59, XP025985519, ISSN: 0002-9378, DOI: 10.1016/J.AJOG.2008.09.195 [retrieved on 2008-12-01]
- Lisa M. Hollier ET AL: "T Helper Cell Cytokine Profiles in Preterm Labor", American Journal of Reproductive Immunology, vol. 52, no. 3, 1 September 2004 (2004-09-01), pages 192-196, XP055205511, ISSN: 8755-8920, DOI: 10.1111/j.1600-0897.2004.00202.x

## Description

### CROSS-REFERENCE TO RELATED APPLICATIONS

The present application claims the benefit of the filing date of U.S. Provisional Application No. 61/267,693 filed December 8, 2009.

### FIELD OF INVENTION

This invention relates to preterm delivery. More specifically, the invention provides methods of using biomarkers for determining preterm delivery risk as defined in the appended claims.

### BACKGROUND

Preterm delivery is one of the most important fetal health problems in the United States today. Approximately one in eight newborns is delivered preterm and the incidence of prematurity has not decreased in the last 20 years. Most preterm babies, if they survive, often have cardiac, neurologic, ophthalmic, and gastrointestinal problems that can extend even beyond childhood, and perhaps lead to adult diseases such as atherosclerosis. Currently, there are few, if any diagnostic biomarkers available that effectively identify women who are going to deliver preterm. Biomarkers that are able to identify these women at risk would be useful in the deployment of prevention strategies. Thus, there is a need to develop novel diagnostics that may identify women who will deliver preterm. Especially, diagnostic biomarkers that may be detected in non-pregnant women or in women during the first trimester.

Marzi, M et al (1996) Clin Exp Immunol. 106: 127-133 report the characterization of type 1 and type 2 cytokine production profile in physiologic and pathologic human pregnancy. Wetta L et al, American Journal of Obstetrics & Gynecology, vol 199, no. 6, 1 December 2008, page S59 (abstract) reports a study of impaired anti-inflammatory response in women with a prior spontaneous preterm birth.

### SUMMARY OF THE INVENTION

In one embodiment, the invention includes ex vivo methods of identifying a non-pregnant woman at risk for preterm delivery, comprising:
(a) stimulating peripheral blood mononuclear cells (PBMCs) from a non-pregnant woman with lipopolysaccharide in the presence of cortisol;
(b) determining the secretion level of one or more cytokines from the stimulated PBMCs, wherein the one or more cytokines comprise one or more of IL-13 and IL-1 Ra;
(c) comparing the secretion level of the one or more cytokines measured in (b) with the secretion level of cytokines from PBMCs from women who delivered at full term stimulated with lipopolysaccharide in the presence of cortisol; and
(d) determining whether the secretion level of at least one of the one or more cytokines comprising one or more of IL-13 and IL-1 Ra from the stimulated PBMCs is higher or lower than the secretion level of the same cytokines in the PBMCs of women who delivered at full term;
   thereby identifying whether the non-pregnant woman is at risk for preterm delivery.

Depending on how much the patient biomarker values are outside the range of the standard controls, the risk of preterm delivery may be determined as "low, medium or high." The biomarkers include but are not limited to IL-13 and/or IL-1RA. The PBMCs may be isolated from a sample of whole blood from the non-pregnant woman prior to stimulation in the presence of cortisol.

In another aspect, the disclosure includes methods of diagnosing susceptibility of preterm delivery in a woman, comprising: obtaining a sample from the woman, determining the expression level of one or more diagnostic biomarkers in the sample, and comparing the expression level of the one or more biomarkers with the expression level of biomarkers from women who delivered fullterm (normal range). If the level of expression of the one or more diagnostic biomarkers in the sample is higher or lower than the level of expression of the same diagnostic biomarkers in the women who delivered fullterm (normal range), then it is indicative that the woman is at risk for preterm delivery. The diagnostic biomarkers can be inflammatory and/or anti-inflammatory cytokines. The diagnostic biomarkers selected include but not limited to IL-10, IL-13 and/or IL-1RA. Biomarker expression levels that are at least one to three times less than the biomarker expression levels of a control are indicative of preterm delivery. The sample can comprise stimulated PBMC/whole blood supernatant (in the presence or absence of cortisol). Alternatively, the sample can comprise serum. The women may be non-pregnant or in the first-trimester of pregnancy.

The present disclosure is also directed to a kit for preterm delivery. The kit is useful for practicing the inventive method of determining the risk of preterm delivery of non-pregnant women. The kit is an assemblage of materials or components, including a diagnostic bioassay of the present disclosure.

The exact nature of the components configured in the kit depends on its intended purpose. For example, some aspects are configured for the purpose of diagnosing susceptibility of women for preterm delivery. In one aspect, the kit is configured particularly for the purpose of determining the risk of preterm delivery of non-pregnant women. In another aspect, the kit is configured particularly for the purpose of diagnosing susceptibility of women for preterm delivery of pregnant or non-pregnant women.

Instructions for use may be included in the kit. "Instructions for use" typically include a tangible expression describing the technique to be employed in using the components of the kit to effect a desired outcome, such as to treat ischemia. Optionally, the kit also contains other useful components, such as, diluents, buffers, pharmaceutically acceptable carriers, syringes, catheters, applicators, pipetting or measuring tools, bandaging materials or other useful paraphernalia as will be readily recognized by those of skill in the art.

The materials or components assembled in the kit can be provided to the practitioner stored in any convenient and suitable ways that preserve their operability and utility. For example the components can be in dissolved, dehydrated, or lyophilized form; they can be provided at room, refrigerated or frozen temperatures. The components are typically contained in suitable packaging material(s). As employed herein, the phrase "packaging material" refers to one or more physical structures used to house the contents of the kit, such as compositions and the like. The packaging material is constructed by well known methods, preferably to provide a sterile, contaminant-free environment. As used herein, the term "package" refers to a suitable solid matrix or material such as glass, plastic, paper, foil, and the like, capable of holding the individual kit components. Thus, for example, a package can be a glass vial used to contain suitable quantities of a composition containing a polyphenol analog. The packaging material generally has an external label which indicates the contents and/or purpose of the kit and/or its components.

### BRIEF DESCRIPTION OF THE FIGURES

Exemplary embodiments are illustrated in referenced figures. It is intended that the embodiments and figures disclosed herein are to be considered illustrative rather than restrictive.

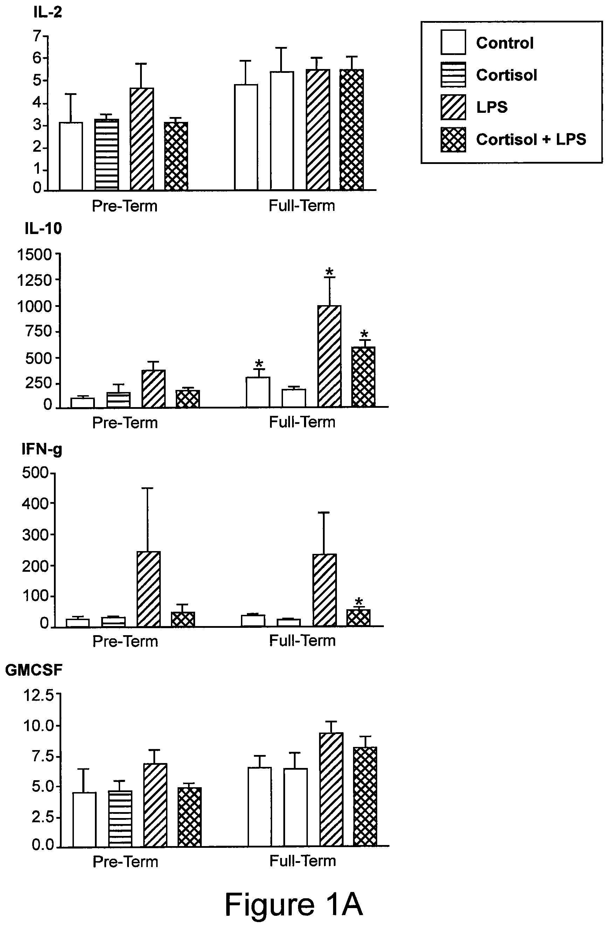

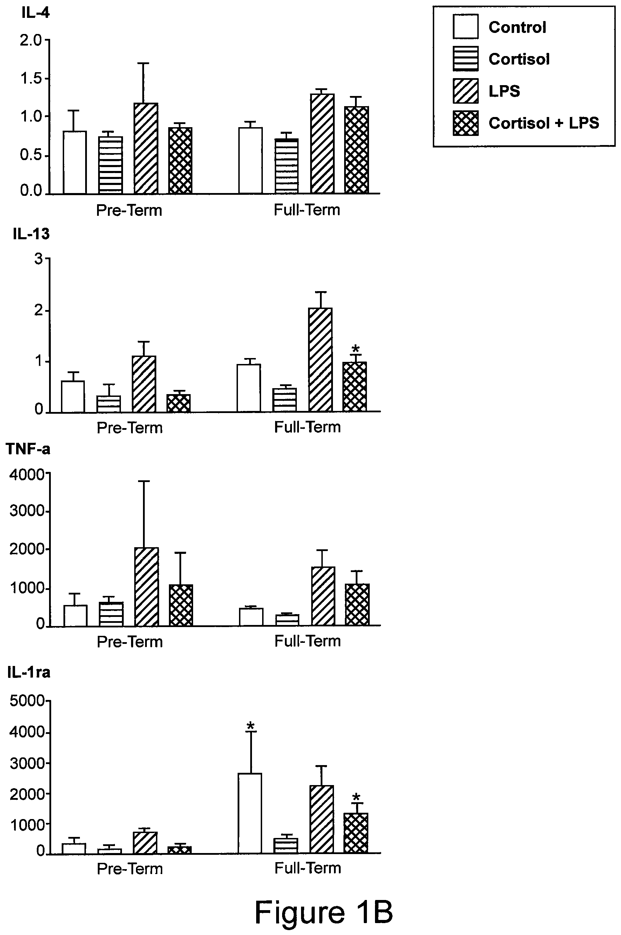

**Figure 1****.** Graphical representation of expression levels of various biomarkers of preterm individuals as compared to full term individuals. Empirical means and standard errors for each cytokine and treatment group are presented.

### DESCRIPTION OF THE INVENTION

Multiple factors lead to preterm delivery; however immune activation is thought be the final affector pathway that leads to preterm rupture of membranes and contractions. Family and genetic studies suggest that prematurity runs in families and history of prior preterm delivery increases the risk of future preterm deliveries. The inventors believed that these observations can be explained by the inherent differences in the immune responses of women who deliver preterm compared with those who deliver full term, and that these differences can be detected even in the non-pregnant state. The inventors believed that by detecting these differences one may be able to identify those women who are at risk to deliver preterm even before they become pregnant.

The inventors are the first to have discovered a biomarker assay that provides physicians with a tool to identify women who are at risk for preterm delivery, even before they become pregnant or while in the first trimester. Identification of women at risk allows the physician to better focus on preventative strategies in these women and improve pregnancy outcome. In one embodiment, the test may be done on small amounts of blood sample obtained from the subject, thus being minimally invasive to the fetus. Furthermore, the test may be repeated multiple times during the course of pregnancy. Thus, it provides dynamic assessment of the preterm delivery risk under the influence of changing environmental/physiologic factors, as wells as requires minimal skills to draw blood as opposed to obtaining amniotic fluid.

One skilled in the art will recognize many methods and materials similar or equivalent to those described herein, which could be used in the practice of the present invention. For purposes of the present diclosure, the following terms are defined below.

As used herein, the term "preterm delivery" refers to a premature birth or conditions associated with a premature birth, including for example, a child delivered before 34 weeks of amenorrhea.

"Biomarker," "diagnostic biomarker," or "preterm delivery biomarker" refers to a molecular indicator that is associated with a particular pathological or physiological state. The "biomarker" as used herein is an indicator for preterm delivery. The indicator can be a cytokine or an immunomodulating agent, including interleukins and interferons. Examples of "biomarkers" include but are not limited IL-2, IL-4, IL-5, IL-8, IL-10, IL-12, IL-13, GM-CSF, IFN-g, and TNF-a. Biomarkers of the present invention include one or more of IL-13 and IL-IRa and optionally further include IL10. A "biomarker" of the present invention may be detected in a sample.

"Sample" or "a biological sample" refers to cells or component parts, or a fraction or portion thereof of body fluids, including but not limited to blood or amniotic cord blood. A "sample" or "biological sample" further refers to plasma, serum, and/or peripheral blood mononuclear cells (PMBC).

In one aspect, the present disclosure provides a method of diagnosing susceptibility for preterm delivery in a non-pregnant woman comprising obtaining a sample from the woman and assaying the sample for the presence or absence of one or more diagnostic biomarkers, where the presence or absence of one or more diagnostic biomarkers is indicative of susceptibility for preterm delivery in the woman. In another aspect the sample comprises PMBC supernatant. In another aspect, the sample comprises whole blood. In another aspect the sample comprises serum. In another aspect, the one or more diagnostic biomarkers comprise a microbial component lipopolysacharide (LPS) or another immune stimulant induced cytokine expression profile. In another aspect, the one or more diagnostic biomarkers comprise inflammation and/or anti-inflammatory cytokines. In another aspect, the diagnostic biomarkers are analyzed in the presence of cortisol. In another aspect, the cortisol concentration is in the range of 1 ug/ml to 500 ug/ml, with a preferred range of 1 ug/ml to 150 ug/ml. In another aspect, the one or more diagnostic biomarkers comprise a low expression of IL-10, IL-13 and/or IL-1RA. In another aspect, the low expression of IL-10, IL-13 and/or IL-1RA comprises a 1 to 3 fold decrease in expression compared to levels ordinarily found in a healthy individual.

There are many techniques readily available in the field for detecting the presence or absence of cytokines or other biomarkers, including protein microarrays. For example, some of the detection paradigms that can be employed to this end include optical methods, electrochemical methods (voltametry and amperometry techniques), atomic force microscopy, and radio frequency methods, e.g., multipolar resonance spectroscopy. Illustrative of optical methods, in addition to microscopy, both confocal and non-confocal, are detection of fluorescence, luminescence, chemiluminescence, absorbance, reflectance, transmittance, and birefringence or refractive index (e.g., surface plasmon resonance, ellipsometry, a resonant mirror method, a grating coupler waveguide method or interferometry).

Similarly, there are any numbers of techniques that may be employed to isolate and/or fractionate biomarkers. For example, a cytokine may be captured using biospecific capture reagents, such as antibodies, aptamers or antibodies that recognize the biomarker and modified forms of it. This method could also result in the capture of protein interactors that are bound to the proteins or that are otherwise recognized by antibodies and that, themselves, can be biomarkers. The biospecific capture reagents may also be bound to a solid phase. Then, the captured proteins can be detected by SELDI mass spectrometry or by eluting the proteins from the capture reagent and detecting the eluted proteins by traditional MALDI or by SELDI. One example of SELDI is called "affinity capture mass spectrometry," or "Surface-Enhanced Affinity Capture" or "SEAC," which involves the use of probes that have a material on the probe surface that captures analytes through a non-covalent affinity interaction (adsorption) between the material and the analyte. Some examples of mass spectrometers are time-of-flight, magnetic sector, quadrupole filter, ion trap, ion cyclotron resonance, electrostatic sector analyzer and hybrids of these.

Alternatively, for example, the presence of biomarkers such as cytokines may be detected using traditional immunoassay techniques. Immunoassay requires biospecific capture reagents, such as antibodies, to capture the analytes. The assay may also be designed to specifically distinguish protein and modified forms of protein, which can be done by employing a sandwich assay in which one antibody captures more than one form and second, distinctly labeled antibodies, specifically bind, and provide distinct detection of, the various forms. Antibodies can be produced by immunizing animals with the biomolecules. Traditional immunoassays may also include sandwich immunoassays including ELISA or fluorescence-based immunoassays, as well as other enzyme immunoassays.

Prior to detection, cytokines may also be fractionated to isolate them from other components in a solution or of blood that may interfere with detection. Fractionation may include platelet isolation from other blood components, sub-cellular fractionation of platelet components and/or fractionation of the desired cytokine from other biomolecules found in platelets using techniques such as chromatography, affinity purification, 1D and 2D mapping, and other methodologies for purification known to those of skill in the art. In one embodiment, a sample is analyzed by means of a biochip. Biochips generally comprise solid substrates and have a generally planar surface, to which a capture reagent (also called an adsorbent or affinity reagent) is attached. Frequently, the surface of a biochip comprises a plurality of addressable locations, each of which has the capture reagent bound there.

Alternatively, for example, the presence of biomarkers such as cytokines may be detected using PCR techniques or flow cytometry. All of the available techniques for cytokine and chemokine detection may be used.

### EXAMPLES

The following examples are provided to better illustrate the claimed invention and are not to be interpreted as limiting the scope of the invention. To the extent that specific materials are mentioned, it is merely for purposes of illustration and is not intended to limit the invention.

### Example 1

### Biomarker Assay

Peripheral blood mononuclear cells (PBMC) from non-pregnant (at least 5-6 years post-partum) women with history of preterm or full term delivery, in addition to the microbial component lipopolysaccharide (LPS)-induced cytokine expression profile were examined. PBMC were separated from whole blood using Ficoll gradient. The cells were counted and equal numbers of cells were plated in 24 well plates. PBMC were treated with cortisol (50 or 300 ug/ml) or media for 1 hour prior to stimulation with LPS (0, 1, or 100 ug/ml) for 24 hours. The PBMC were lysed and the supernatant was examined for inflammatory and anti-inflammatory cytokine expression by using Bioplex technology (Bio-rad). The inventors found that during the non-pregnant state IL10, IL13 and IL1Ra expression was lower in the PBMC obtained from women whom had previous preterm delivery, and that those biomarkers may be measured to identify women who were at risk for preterm delivery in the future.

In this study two patients were preterm and four were full term. There were 9 samples per patient (3x3 design) and all samples from each patient were analyzed twice for cytokine concentrations with the exception of one full term subject who only had one analysis of each sample. As a result there are a total of 99 observations for each cytokine measured. The assay simultaneously measured concentrations of 11 inflammatory markers: IL-1ra (ILl-receptor antagonist), IL-2, IL-4, IL-5, IL-8, IL-10, IL-12, IL-13, GM-CSF, IFN-g, and TNF-a.

### Example 2

### Establishment of cut off points

Many samples were above or below the detection for some of the endpoints. Cut off points were established for each cytokine measured **(Table 1).** If a sample was out of the range of detection, a default value was assigned to the sample as indicated below.

**Table 1. The establishment of cut off points for out of range values.**

| Cytokine | Limit of Detection | Out of Range Set Value | Fraction of Samples out of Detection Range |
|---|---|---|---|
| IL-1 ra | *all samples within levels of detection* | *n*/*a* | 0/99 |
| IL-2 | LL = 0.50 | 0.25 | 10/99 |
| IL-4 | LL = 0.14 | 0.08 | 6/99 |
| IL-5 | LL = 0.90 | 0.5 | 82/99 |
| IL-8 | UL = 95,000 | 100,000 | 79/99 |
| IL-10 | *all samples within levels of detection* | *n*/*a* | 0/99 |
| IL-12 | LL = 0.33 | 0.15 | 19/99 |
| IL-13 | LL = 0.17 | 0.08 | 8/99 |
| GM-CSF | LL = 1.18 | 0.60 | 11/99 |
| IFN-g | LL = 1.21 | 0.60 | 5/99 |
| TNF-a | *all samples within levels of detection* | *n*/*a* | 0/99 |

| | | | |
|---|---|---|---|
| **LL = Lower Limit; UL = Upper Limit** | | | |

All samples that were below the range of detection for IL-2, IL-4, IL-13, and IFN-g were from the two subjects with a history of pre-term delivery. Of the 11 samples assayed that were below detection limits for GM-CSF, only 1 was from a subject with a full-term delivery while the rest were from subjects with histories of pre-term deliveries.

### Example 3

### Statistical analysis

Empirical means and standard errors for each cytokine and treatment group are presented herein. Here, the averages of replicate measures were calculated first for each subject. The mean and SEM was then taken across each treatment group.

All raw data was tested via the Kolmogorov-Smirnov test to determine if the data followed a normal distribution. Log-transformations were performed for all data found to have a non-normal distribution prior to further statistical analysis. For each cytokine, a mixed effects model was used to examine all data for significant effects with the outcome variable as the cytokine concentration; the fixed predictor variables as LPS concentration, cortisol concentration, and delivery status (pre- or full-term); and the random effect due to the replicate data points. To test the interaction of the three fixed predictor variables on cytokine concentration, both first and second level interactions ware added to each cytokine model. Post-hoc testing was performed using a Student's t-test.

### Example 4

### Cortisol and LPS concentrations

All 99 observations were initially used in the analysis to first determine which concentrations of LPS and cortisol induced the most robust effect. For all cytokines measured there was no significant difference between the effects of 1 ug/ml and 100 ug/ml LPS. For several cytokines (IL-lra, IL-10, and IL-13), 300 ug/ml cortisol had a more significant effect to suppress cytokine levels than 50 ug/ml.

As a result of the findings, the inventors focused on the data with 0 or 300 ug/ml of cortisol pretreatment crossed with 0 or 1 ug/ml LPS. This results in 4 samples per subject, each analyzed twice (with exception of the samples from the full term subject who only had one analysis of each sample). With 8 measurements per subject (with the exception of the full term subject who only has 4 measurements) this results in a data set with 44 measurements for each of the cytokines.

### Example 5

### Analysis of testing for cytokine differences between groups

Using the same mixed model regression, estimated group means and standard errors were calculated. Differences between preterm and full-term pregnancies were considered significant where p < 0.10.

Based on the results of the statistical modeling, cortisol did suppress secretion of IL-13 and IL-lra; LPS increased secretion of IL-4, IL-10, IL-13, TNF-a, and IL-lra; and women who had preterm deliveries had overall lower secretion levels of IL-10, IL-13, and IL-lra **(Table 2).**

**Table 2: Global effects of cortisol, LPS, and premature delivery on cytokine secretion levels in PBMC based on results of mixed modeling.**

| Cytokine | Cortisol Effect | LPS Effect | Pre-term Effect |
|---|---|---|---|
| IL-2 | n/c, p = .80 | n/c, p = .50 | n/c, p = .17 |
| IL-4 | n/c, p = .26 | increase, p = .07 | n/c, p = .59 |
| IL-10 | n/c, p = .12 | increase, p = .01 | decrease, p = .01 |
| IL-13 | decrease, p = .01 | increase, p = .04 | decrease, p = .02 |
| IFN-g | n/c, p = .10 | n/c, p = .17 | n/c, p = .22 |
| TNF-a | n/c, p = .41 | increase, p = .04 | n/c, p = .75 |
| GM-CSF | n/c, p = .51 | n/c, p = .19 | n/c, p = .17 |
| IL-1ra | decrease, p = .03 | increase, p = .09 | decrease, p = .01 |

| | | | |
|---|---|---|---|
| **n/c = No Change** | | | |

Women who had pre-term deliveries had lower baseline IL-10, 1L13 and IL-IRa expression compared to those who delivered full term. There were no differences in cytokine production between LPS-stimulated PBMC from preterm delivering women when compared with that from women with full-term deliveries. In other words, adjusting for unstimulated secretion levels, the concentration of cytokines released from LPS stimulation was no different between the two groups of women. In the presence of cortisol PBMC from women who had pre-term deliveries produced lower IL-13 and IFN-g, expression when compared with that from women with full-term deliveries.

### References

Hutzal CE, Boyle EM, Kenyon SL, Nash JV, Winsor 5, Taylor DJ, Kirpalani H. Use of antibiotics for the treatment of preterm parturition and prevention of neonatal morbidity: a metaanalysis. Am J Obstet Gynecol. 2008 Dec; I 99(6):620.e1-8. Epub 2008 Oct 30.
Swamy GK, Ostbye T, Skjaerven R. Association of preterm birth with long-term survival, reproduction, and next-generation preterm birth. JAMA. 2008 Mar 26;299(12):1429-36. Erratum in: JAMA. 2008 Jul 9;300(2):170-1.
DeFranco E, Teramo K, Muglia L. Genetic influences on preterm birth. Semin Reprod Med. 2007 Jan;25(1):40-51.
Plunkett J, Muglia LJ. Genetic contributions to preterm birth: implications from epidemiological and genetic association studies. Ann Med. 2008;40(3):167-95.
Goldenberg RL, Culhane IF, lams JD, Romero R. Epidemiology and causes of preterm birth. Lancet. 2008 Jan 5;371(9606):75-84.
Blank V, Hirsch E, Challis JR, Romero R, Lye Si. Cytokine signaling, inflammation, innate immunity and preterm labour - a workshop report. Placenta. 2008 Mar;29 Suppl A:S102-4.
Mazaki-Tovi S, Romero R, Kusanovic JP, Erez 0, Pineles BL, Gotsch F, Mittal P, Than NG, Espinoza J, Hassan SS. Recurrent preterm birth. Semin Perinatol. 2007 Jun;31(3):142-58.
Romero R, Espinoza J, Goncalves LF, Kusanovic JP, Friel L, Hassan S. The role of inflammation and infection in preterm birth. Semin Reprod Med. 2007 Jan;25(I):21-39.
Romero R, Espinoza J, Gonsalves LF, Kusanovic JP, Friel LA, Nien JK. Inflammation in preterm and term labour and delivery. Semin Fetal Neonatal Med. 2006 Oct;11(5):317-26.
Luu TM, Ment LR, Schneider KC, Katz KH, Allan WC, Vohr BR. Lasting effects of preterm birth and neonatal brain hemorrhage at 12 years of age. Pediatrics. 2009 Mar;123(3):1037-44. -Morse SB, Zheng H, Tang Y, Roth J. Early school-age outcomes of late preterm infants. Pediatrics. 2009 Apr;123(4):e622-9.
Kamholz KL, Cole CH, Gray JE, Zupancic JA. Cost-effectiveness of early treatment for retinopathy of prematurity. Pediatrics. 2009 Jan;123(1):262-9.
Limperopoulos C, Bassan }I, Sullivan NR, Soul JS, Robertson RL Jr, Moore M, Ringer SA, Volpe JJ, du Plessis AJ. Positive screening for autism in ex-preterm infants: prevalence and risk factors. Pediatrics, 2008 Apr;121(4):758-65.
Jacobs SE, O'Brien K, Inwood 5, Kelly EN, Whyte HE. Outcome of infants 23-26 weeks of gestation pre and post surfactant. Acta Paediatr 2000, 89(8):959-965.
Hack M, Flannery DJ, Schluchter M, et al. Outcomes in young adulthood for very-low-birth-weight infants. N EngI Med 2002, 346(3):149-157.
Gent, MR, Gerber S, Nesin M, Witkin SS. Polymorphism in the interleukin-1 gene complex and spontaneous preterm delivery. Am J Obstet Gynecol. 2002 Jul;187(1):157-63.
Peltier MR. Immunology of term and preterm labor. Reprod Biol Endocrinol. 2003 Dec 2;1:122.
Kalish RB, Vardhana S, Gupta M, Perni SC, Witkin SS, Interleukin-4 and -10 gene polymorphisms and spontaneous preterm birth in multifetal gestations. Am 1 Obstet Gynecol. 2004 Mar;190(3):702-6.
Gotsch F, Romero R, Kusanovic JP, Erex 0, Espinoza J, Kim CJ, Vaisbuch E, Than NG, Mazaki-Tovi S Chaiworapongsa T, Mazor M, Yoon BH, Edwin S, Gomez R, Mittal P, Hassan SS, Sharma S. The antiinflammatory limb of the immune response in preterm labor, intra-amniotic infection/inflammation, and spontaneous parturition at term: a role for interleukin-10. J Matern Fetal Neonatal Med. 2008 Aug;2 I (8):529-47.
Berghella V, Hayes E, Visintine J, Baxter JK. Fetal fibronectin testing for reducing the risk of preterm birth. Cochrane Database Syst Rev. 2008 Oct 8;(4):CD006843.
Herbst A, Nilsson C. Diagnosis of early preterm labour. BJOG. 2006 Dec;113 Suppl 3:60-7. Review. Erratum in: BJOG. 2008 Apr;115(5):674-5.
Lockwood CJ, Senyei AE, Dische MR. Casal D, Shah KD, Thung SN, Jones L. Deligdisch L, Garite D. Fetal fibronectin in cervical and vaginal secretions as a predictor of preterm delivery. N Engl J Med. 1991 Sep 5;325(10):669-74.
Honest H, Bachmann LM, Gupta JK, Kleijnen .1, Khan KS. Accuracy of cervicovaginal fetal fibronectin test in predicting risk of spontaneous preterm birth: systematic review. BMJ. 2002 Aug 10;325(7359):301.
Most 0, Langer 0, Kerner R, David GB, Calderon!. Can myometrial electrical activity identify patients in preterm labor? Am J Obstet Gynecol. 2008 Oct;199(4):378.e1-6.
Menon R. Camargo MC, Thorsen P, Lombardi SJ, Fortunate Si. Amniotic fluid interleukin-6 increase is an indicator of spontaneous preterm birth in white but not black Americans. Am .1 Obstet Gynecol. 2008 Jan;198(1):77,e1-7.
Vogel 1, Goepfert AR, Thorsen P, Skogstrand K, Hougaard DM, Curry AH, Cliver S, Andrews WW. Early second-trimester inflammatory markers and short cervical length and the risk of recurrent preterm birth. J Reprod immune!. 2007 Oct;75(2):133-40. Epub 2007 Apr 17.
Kim KW, Romero R, Park HS, Park CW, Shim SS, Jun JK, Yoon BH. A rapid matrix metalloproteinase-8 bedside test for the detection of intraamniotic inflammation in women with preterm premature rupture of membranes. Am .1 Obstet Gynecol. 2007 Sep;197(3):292.e1 -5.
Spong CY. Prediction and prevention of recurrent spontaneous preterm birth. Obstet Gynecol. 2007 Aug;110(2 Pt 1):405-15. Review.
Ghosh G. Breborowicz A, Brazen M, Maczkiewicz M, Kobelski M, Dubiel M, Gudmundsson S. Evaluation of third trimester uterine artery flow velocity indices in relationship to perinatal complications.1 Matern Fetal Neonatal Med. 2006 Sep;19(9):551-5.
Leitich H. Secondary predictors of preterm labour. BJOG. 2005 Mail] 12 Suppl 1:48-50. Review.
Challis JR. Maternal corticotropin-releasing hormone, fetal growth, and preterm birth. Am J Obstet Gynecol. 2004 Oct; 191(4):1059-60.
Heine RP, McGregor JA, Goodwin TM, Anal R, Hayashi RH, Robertson PA, Varner MW. Serial salivary estriol to detect an increased risk of preterm birth. Obstet Gynecol. 2000 Oct;96(4):490-DWI 13662752vl 0067789-254 PRO

## Claims

1. An ex vivo method of identifying a non-pregnant woman at risk for preterm delivery, comprising:
(a) stimulating peripheral blood mononuclear cells (PBMCs) from a non-pregnant woman with lipopolysaccharide in the presence of cortisol;
(b) determining the secretion level of one or more cytokines from the stimulated PBMCs, wherein the one or more cytokines comprise one or more of IL-13 and IL-1Ra;
(c) comparing the secretion level of the one or more cytokines measured in (b) with the secretion level of cytokines from PBMCs from women who delivered at full term stimulated with lipopolysaccharide in the presence of cortisol; and
(d) determining whether the secretion level of at least one of the one or more cytokines comprising one or more of IL-13 and IL-1Ra from the stimulated PBMCs is higher or lower than the secretion level of the same cytokines in the PBMCs of women who delivered at full term;
thereby identifying whether the non-pregnant woman is at risk for preterm delivery.

2. The method according to claim 1, in which step (b) comprises determining the secretion level of IL-13 from the stimulated PBMCs.

3. The method according to claim 1, in which step (b) further comprises determining the secretion level of IL-10 from the stimulated PBMCs.

4. The method according to claim 2, in which step (b) further comprises determining the secretion levels of IL-1 Ra and IL-10 from the stimulated PBMCs.

5. The method according to claim 1, in which step (b) comprises determining the secretion level of IL-1 Ra from the stimulated PBMCs.

6. The method according to any one of claims 1 to 5, wherein the secretion level shows a one- to three-fold decrease as compared to the secretion level of the same cytokine in the PBMCs of the women who delivered at full term.

7. The method according to any one of claims 1 to 6, wherein the secretion level is determined using an immunoassay.

8. The method according to any one of claims 1 to 7, wherein the PBMCs are isolated from a sample of whole blood from the non-pregnant woman prior to stimulation.

## Patentansprüche

1. Ex-Vivo-Verfahren zur Identifizierung einer nicht schwangeren Frau mit einem Risiko einer vorzeitigen Entbindung, umfassend:
(a) Stimulieren von mononukleären Zellen des peripheren Bluts (PBMCs) von einer nicht schwangeren Frau mit Lipopolysaccharid in der Gegenwart von Cortisol;
(b) Bestimmen des Sekretionsniveaus eines oder mehrerer Zytokine aus den stimulierten PBMCs, wobei das eine oder die mehreren Zytokine eines oder mehrere von IL-13 und IL-1Ra umfassen;
(c) Vergleichen des in (b) gemessenen Sekretionsniveaus des einen oder der mehreren Zytokine mit dem Sekretionsniveau von Zytokinen aus PBMCs von Frauen, die termingerecht entbanden, stimuliert mit Lipopolysaccharid in der Gegenwart von Cortisol; und
(d) Bestimmen, ob das Sekretionsniveau von zumindest einem des einen oder der mehreren Zytokine, umfassend eines oder mehrere von IL-13 und IL-1Ra, aus den stimulierten PBMCs höher oder niedriger ist als das Sekretionsniveau der gleichen Zytokine in den PBMCs von Frauen, die termingerecht entbanden;
wodurch identifiziert wird, ob die nicht schwangere Frau einem Risiko einer vorzeitigen Entbindung ausgesetzt ist.

2. Verfahren nach Anspruch 1, wobei Schritt (b) das Bestimmen des Sekretionsniveaus von IL-13 aus den stimulierten PBMCs umfasst.

3. Verfahren nach Anspruch 1, wobei Schritt (b) ferner das Bestimmen des Sekretionsniveaus von IL-10 aus den stimulierten PBMCs umfasst.

4. Verfahren nach Anspruch 2, wobei Schritt (b) ferner das Bestimmen der Sekretionsniveaus von IL-1 Ra und IL-10 aus den stimulierten PBMCs umfasst.

5. Verfahren nach Anspruch 1, wobei Schritt (b) das Bestimmen des Sekretionsniveaus von IL-1 Ra aus den stimulierten PBMCs umfasst.

6. Verfahren nach einem der Ansprüche 1 bis 5, wobei das Sekretionsniveau einen ein- bis dreifachen Rückgang im Vergleich zu dem Sekretionsniveau des gleichen Zytokins in den PBMCs der Frauen, die termingerecht entbanden, zeigt.

7. Verfahren nach einem der Ansprüche 1 bis 6, wobei das Sekretionsniveau unter Anwendung eines Immunoassays bestimmt wird.

8. Verfahren nach einem der Ansprüche 1 bis 7, wobei die PBMCs vor der Stimulation von einer Vollblutprobe der nicht schwangeren Frau isoliert werden.

## Revendications

1. Procédé ex vivo d'identification d'une femme non enceinte présentant un risque d'accouchement avant terme, comprenant :
(a) la stimulation des cellules mononucléaires sanguines périphériques (PBMC) provenant d'une femme non enceinte avec un lipopolysaccharide en présence de cortisol ;
(b) la détermination du niveau de sécrétion d'une ou plusieurs cytokines provenant des PBMC stimulées, lesdites une ou plusieurs cytokines comprenant une ou plusieurs interleukines parmi IL-13 et IL-IRa ;
(c) la comparaison du niveau de sécrétion desdites une ou plusieurs cytokines mesurées en (b) avec le niveau de sécrétion des cytokines provenant des PBMC de femmes qui ont accouché à terme stimulées avec un lipopolysaccharide en présence de cortisol ; et
(d) la détermination pour savoir si le niveau de sécrétion d'au moins l'une desdites une ou plusieurs cytokines comprenant une ou plusieurs interleukines parmi IL-13 et IL-IRa provenant des PBMC stimulées est supérieur ou inférieur au niveau de sécrétion des mêmes cytokines dans les PBMC de femmes qui ont accouché à terme ;
ce qui permet d'identifier si la femme non enceinte présente un risque d'accouchement avant terme.

2. Procédé selon la revendication 1, dans lequel l'étape (b) comprend la détermination du niveau de sécrétion d'IL-13 à partir des PBMC stimulées.

3. Procédé selon la revendication 1, dans lequel l'étape (b) comprend en outre la détermination du niveau de sécrétion d'IL-10 à partir des PBMC stimulées.

4. Procédé selon la revendication 2, dans lequel l'étape (b) comprend en outre la détermination des niveaux de sécrétion d'IL-IRa et d'IL-10 à partir des PBMC stimulées.

5. Procédé selon la revendication 1, dans lequel l'étape (b) comprend la détermination du niveau de sécrétion d'IL-IRa à partir des PBMC stimulées.

6. Procédé selon l'une quelconque des revendications 1 à 5, ledit niveau de sécrétion présentant une réduction d'un facteur 1 à 3 par rapport au niveau de sécrétion de la même cytokine dans les PBMC des femmes qui ont accouché à terme.

7. Procédé selon l'une quelconque des revendications 1 à 6, ledit niveau de sécrétion étant déterminé à l'aide d'un dosage immunologique.

8. Procédé selon l'une quelconque des revendications 1 à 7, lesdites PBMC étant isolées à partir d'un échantillon de sang complet provenant d'une femme non enceinte avant stimulation.
